# EUROPEAN PATENT APPLICATION

(11) **EP 2 843 041 A1**
(43) Date of publication of application: **04.03.2015**
(21) Application number: 13781861.3
(22) Date of filing: 17.04.2013
(51) Int. Cl.: C12N 5/02, C12N 5/074

(54) **STEM CELL CULTURE MEDIUM AND METHOD FOR CULTURING STEM CELLS USING SAME**

(30) Priority: 24.04.2012 KR 20120042671
(71) Applicant: Samsung Life Public Welfare Foundation, Yongsan-gu, Seoul 140-863 (KR)
(72) Inventor: NAM, Do Hyun, Sungnam-si Gyeonggi-do 436-922 (KR)
(74) Representative: Hiebl, Inge Elisabeth
(86) International application number: PCT/KR2013/003218
(87) International publication number: WO 2013/162199

(57) **Abstract**

The present invention relates to a stem cell culture medium which can be substituted for a conventional stem cell culture medium containing the heterologous protein fetal bovine serum, and more particularly to a stem cell culture medium containing a basal medium and a knockout serum replacement and a method of culturing stem cells using the same. According to the invention, a high purity of stem cells having a reduced ability to spontaneously differentiate can be obtained without having to use the heterologous protein fetal bovine serum and expensive growth factors (EGF and bFGF), and thus the efficacy of stem cell therapy can be significantly increased.

## Description

### TECHNICAL FIELD

The present invention relates to a medium for culturing stem cells, and more particularly to a stem cell culture medium containing a knockout serum replacement (KoSR) and a method of culturing stem cells using the same.

According to the present invention, a high purity of stem cells required for treatment can be obtained even at low costs without the risk of contamination by heterologous protein, and thus can be effectively used in stem cell therapy.

### BACKGROUND ART

21^{th} biotechnology presents the possibility of new solutions to the food, environment and health issues with the ultimate goal of promoting human welfare. In recent years, the technology of using stem cells has been considered as a new way to treat incurable diseases. Formerly, organ transplantation, gene therapy, etc., were presented for the treatment of incurable human diseases, but these have not been efficiently used due to immune rejection, the short supply of organs, the insufficient development of vectors, and the insufficient knowledge of disease genes.

For this reason, with increasing interests in stem cell studies, it has been recognized that totipotent stem cells having the ability to form all the organs by proliferation and differentiation can not only treat most of diseases but also fundamentally heal organ injuries. Stem cells refer to cells having not only self-replication ability but also the ability to differentiate into at least two types of cells, and themselves fall into totipotent stem cells, pluripotent stem cells, and multipotent stem cells. In addition, many scientists have suggested the applicability of stem cells for the regeneration of all the organs and the treatment of incurable diseases, including Parkinson's disease, various cancers, diabetes and spinal damages.

Particularly, neural stem cells have the ability to self-replicate and differentiate into neurons, astrocytes and oligodendrocytes, which make up the central nervous system. Thus, fundamental studies on the use of such neural stem cells for the proliferation and differentiation of stem cells and the development of the central nervous system have increased, and interest in the new possibility of using the biological characteristics of neural stem cells to perform cell and gene therapy in diseases of the nervous system which does not regenerate once damaged also has increased. In addition, methods of establishing autologous adult neural stem cells by the culture of human adult brain tissue can be free from ethical concerns compared to other methods that use embryonic stem cells or fetal brain tissue. Also, these methods enable patient-tailored cell therapy that can be an alternative to overcome the limitation of conventional cell therapy.

In order to utilize these advantages, many researchers have attempted to culture human adult neural stem cells, but studies thereon are at a standstill, because human adult neural stem cells are difficult to culture *in vitro* and their ability to proliferate is also limited. It is an established theory in stem cell biology that these stem cells require a specific cellular microenvironment or niche in their culture. Reported culture techniques for selectively culturing neural stem cells include a neurosphere forming method, a low-density culture method, a high-density culture method, etc. In addition, known methods include methods of culturing embryonic stem cell-, fetal brain tissue- or human adult brain tissue-derived adult neural stem cells in the presence of growth factors.

The present inventors previously filed a patent for an invention relating to a method of culturing human adult neural stem cells using a low concentration of fetal bovine serum (FBS) (see Korean Patent Application Nos. 2010-0010116 and 2010-0010117). However, a medium or a culture method has not yet been known which can culture human adult neural stem cells without an expensive growth factor and fetal bovine serum.

The present inventors established a technique for primary culture of adult neural stem cells and a technique for mass culture of adult neural stem cells (see Korean Patent Application Nos. 2010-0010116 and 2010-0010117 and PCT International Patent Application No. PCT/KR2011/000730). Based on these techniques, the present inventors have investigated a cell culture medium, which contains no expensive growth factors while making it possible to avoid contamination caused by heterologous protein, and thus can be clinically applied.

Accordingly, the present inventors have cultured stem cells in a medium containing a knockout serum replacement (KoSR), and as a result, have found that KoSR can be used as a substitute for serum, thereby completing the present invention.

### DISCLOSURE OF INVENTION

It is an object of the present invention to provide a stem cell culture medium which makes it possible to obtain a high purity of stem cells even at low costs without being exposed to heterologous protein.

Another object of the present invention is to provide a method of culturing stem cells using the above culture medium.

To achieve the above objects, the present invention provides a stem cell culture medium containing a basal medium and a knockout serum replacement (KoSR).

Other features and embodiments of the present invention will be more apparent from the following detailed descriptions and the appended claims.

### BRIEF DESCRIPTION OF THE DRAWINGS

FIG. 1A shows the results of flow cytometry (FACS) for the expression of markers specific for neural stem cell-specific markers (Nestin, Sox1, and Sox2), a glial cell-specific marker (CD44), an astrocyte-specific marker (GFAP) and a neuron-specific marker (Boublecortin; DCX) in adult neural stem cells obtained by the primary culture of adult neural stem cells derived from human temporal lobe brain tissue.
FIG. 1B shows the results of immunocytochemistry for the expression of marker proteins (Nestin, Sox2, CD133, and Hes3) specific for adult neural stem cells derived from human temporal lobe brain tissue.
FIG. 2A schematically shows a process for culturing human temporal lobe brain tissue-derived adult neural stem cells using a knockout serum replacement (KoSR).
FIG. 2B shows photographs of human adult neural stem cells cultured in each of a neural stem cell culture medium (a), which contains FBS (fetal bovine serum) and a growth factor, and the inventive culture medium (b) containing a knockout serum replacement (KoSR).
FIG. 3A shows the results of immunocytochemistry for the expression patterns of a neural stem cell-specific marker protein (Nestin), an astrocyte-specific marker protein (GFAP), an oligodendrocyte-specific marker (04) and a neuron-specific marker (Tuj-1) in cells (#1) in the inventive media containing the knockout serum replacement at various concentrations (1%, 10%, and 20%). As a control, a medium containing 0.5% FBS without containing the knockout serum replacement (KoSR) was used.
FIG. 3B shows the results of flow cytometry (FACS) for the expression of the neuron-specific marker Tuj-1 in the cells (#1) shown in FIG. 3A.
FIG. 3C shows the results of quantification of the FACS analysis results shown in FIG. 3B.
Fig. 4A shows the results of immunocytochemistry for the expression patterns of a neural stem cell-specific marker protein (Nestin), an astrocyte-specific marker protein (GFAP), an oligodendrocyte-specific marker (04) and a neuron-specific marker (Tuj-1) in cells (#2) cultured in the inventive media containing the knockout serum replacement (KoSR) at various concentrations (0%: control, 1%, 10%, and 20%).
FIG. 4B shows the results of flow cytometry (FACS) for the expression of the neuron-specific marker Tuj-1 in the cells (#2) shown in FIG. 4A.
FIG. 4C shows the results of quantification of the FACS analysis results shown in FIG. 4B.

### BEST MODE FOR CARRYING OUT THE INVENTION

In one aspect, the present invention is directed to a stem cell culture medium containing a basal medium and a knockout serum replacement (KoSR).

As used herein, the expression "culture medium containing a(the) knockout serum replacement" refers to a culture medium containing the knockout serum replacement (KoSR) in a basal medium. The culture medium according to the present invention does not contain growth factors, such as fetal bovine serum (FBS), bFGF or EGF, which have been used in the culture of stem cells.

Fetal bovine serum that has been frequently used in cell and tissue culture is known as a complex medium containing a number of any unknown factors in addition to hormones, growth factors and vitamins, and there is a report that fetal bovine serum may be the cause of several abnormal phenomena which appear in calves during fetal development after embryo transplantation and immediately after birth (see Sang-Ki Lee et al., "Effects of Fetal Bovine Serum and Its Replacements (BSA and PVA) on Development of Cloned Embryos"). Thus, when human adult or embryonic stem cells are cultured using a FBS-containing medium, the stem cells are exposed to heterologous proteins, and thus the clinical application of the stem cells cultured in this medium is problematic in terms of safety. In addition, the differentiation, expression, etc. of stem cells can depend on the components of medium used, and thus it is not preferable to use, as the component of culture medium, FBS containing a number of unknown factors. Further, growth factors such as EGF and bFGF are expensive, and thus the mass culture of stem cells is difficult in terms of costs.

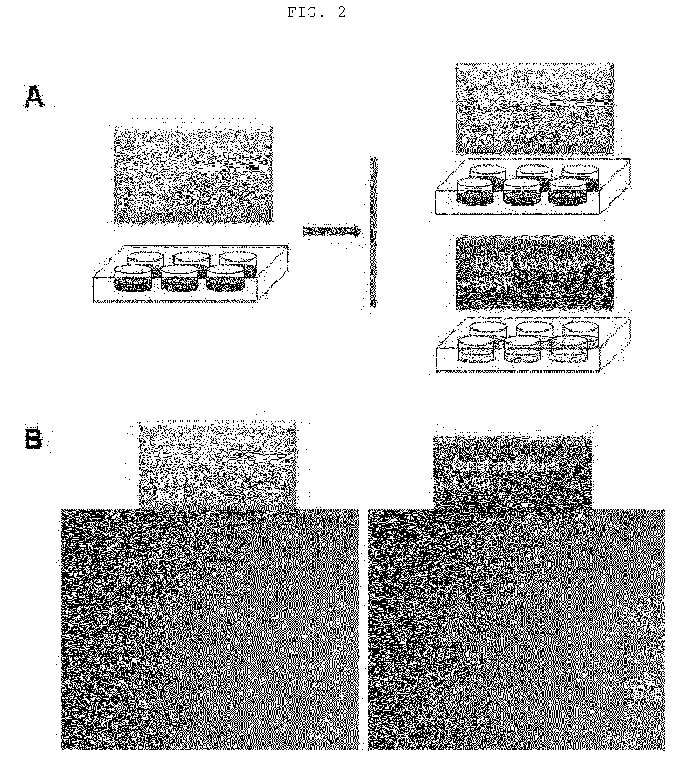

Although the culture medium according to the present invention does not contain FBS and growth factors (EGF, bFGF, etc.), human adult stem cells cultured in the culture medium are cultured in a manner similar to culture in conventional media (see FIG. 2B). Thus, the culture medium according to the present invention can be substituted for conventional culture media containing FBS and high concentrations of expensive growth factors.

The knockout serum replacement (KoSR) that is used in the present invention may contain amino acids (glycine, L-histidine, L-isoleucine, L-methionine, L-phenylalanine, L-proline, L-hydroxyproline, L-serine, L-threonine, L-tryptophan, L-tyrosine, L-valine), vitamins/antioxidants (thiamine, reduced-glutathione, ascorbic acid 2-PO₄), microelements (Ag⁺, Al³⁺, Ba²⁺, Cd²⁺, Co²⁺, Cr³⁺, Ge⁴⁺, Se⁴⁺, Br⁻, I⁻, F⁻, Mn²⁺, Si⁴⁺, V⁵⁺, Mo⁶⁺, Ni²⁺, Rb⁺, Sn²⁺, Zr⁴⁺), proteins (transferrin (iron-saturated), insulin, and lipid-rich albumin (AlbuMAX) (Albumin-associated lipids regulate human embryonic stem cell self-renewal, Francesc R. etc., PLoS ONE 3(1): e1384). The knockout serum replacement used in the present invention is a product commercially available from Invitrogen (Catalog number: 10828-028). The knockout serum replacement may be contained in the medium of the present invention in an amount of 1-20%.

The basal medium that is used in the present invention is a conventional basal medium that is used in the culture of stem cells and known to be suitable for the culture of stem cells in the art. Examples of the basal medium that is used in the present invention include DMEM, MEM, K-SFM media and the like. Preferably, a serum-free medium may be used as the basal medium, and most preferably, a mixture of DMEM: F12 media, B27 supplement and antibiotics may be used. In the present invention, penicillin and streptomycin (Invitrogen) were used as antibiotics, but are not limited thereto, and any antibiotics may be used without limitation in the present invention, as long as they are generally used in the art.

In addition, the basal medium may be supplemented with known additives that promote the proliferation of the undifferentiated phenotype of human neural stem cells while inhibiting the differentiation of the cells. In addition, the medium may contain a neutral buffer (such as phosphate and/or high concentration bicarbonate) in isotonic solution; a protein nutrient (e.g., essential and non-essential amino acids such as glutamine). Furthermore, it may contain lipids (fatty acids, cholesterol, an HDL or LDL extract of serum) and other ingredients found in most stock media of this kind (such as insulin or transferrin, nucleosides or nucleotides, pyruvate, a sugar source such as glucose, selenium in any ionized form or salt, a glucocorticoid such as hydrocortisone and/or a reducing agent such as β-mercaptoethanol). In addition, it may contain an anti-clumping agent, such as a product commercially available from Invitrogen (Cat #0010057AE), in order to prevent cells from adhering to each other, adhering to a vessel wall, or forming clusters that are too big.

As used herein, the term "stem cells" refers to undifferentiated cells that can differentiate into cells constituting tissues and that differentiate into specific cells under specific differentiation stimulus (environment). Unlike differentiated cells with arrested cell division, stem cells retain the capability of self-renewal through cell division, and thus can proliferate (expand). Moreover, stem cells differentiate into specific cells when differentiation stimuli is applied thereto, and they can also differentiate into different cells under different environments or differentiation stimulus, indicating that stem cells have plasticity in differentiation. Such stem cells can be divided, according to the developmental origin thereof, into embryonic stem cells and adult stem cells. In the present invention, it is preferred to use adult stem cells rather than embryonic stem cells that raise serious biological, ethical and legal questions limiting the clinical application thereof.

As used herein, the term "adult stem cell" refers to stem cells extracted from adult body tissues, which are immediately before differentiating into the cells of a specific organ. Adult stem cells are difficult to proliferate and have a strong tendency to differentiate easily, but can differentiate into tissue-specific progenitor cells in the human body. Adult stem cells can differentiate into cells having various characteristics and have the capability to produce replacement cells for various tissues and organs, including heart, pancreas, nerve tissue, muscle and cartilage. A method for isolating adult stem cells from various human tissues may be performed using a conventional method known in the art, which is suitable for each tissue. For example, a method may be used which comprises treating a collected specific tissue with trypsin solution and/or collagenase to isolate single cells, culturing the single cells in a medium supplemented with suitable amounts of growth factors (e.g., bFGF, EGF, etc.), and isolating adult stem cells from the culture by FACS or according to growth rate. Preferably, adult neural stem cells or neural crest stem cells (NCSCs) may be used in the present invention.

As used herein, the term "neural stem cells" refers to cells that are capable of undergoing greater than 20-30 cell divisions and maintain the potency to generate both neurons and glia. Preferably, such cells are capable of undergoing greater than 40, more preferably greater than 50, most preferably unlimited cell divisions. The neural stem cells are by definition multipotent, i.e. they are capable of differentiating into a number of neural cell types (e.g. neurons/glia). The neural stem cells can be obtained by primary culture of the tissues of the central nervous system (CNS) and the peripheral nervous system (PNS) and differentiate into glial lineage and neural lineage cells under specific conditions (Sally Temple et al. 2001). As used herein, the term "neural crest stem cells (NCSCs)" refers to stem cells that temporally appear during the early embryonic developmental process, and these cells are also multipotent stem cells.

It is possible to obtain neural stem cells from various sources. For example, neural stem cells can be derived from human adult brain tissue, wherein the brain may be any one selected from the group consisting of cerebrum, diencephalon, mesencephalon, cerebellum, medulla oblongata, pons, and spinal cord. Preferably, neural stem cells can be derived from cerebral tissue, such as temporal lobe tissue or hippocampus tissue. Human neural stem cells can be purchased from commercially available sources, and preferably, they can be produced by culturing cells, obtained from human adult brain tissue, in a medium containing neural stem cell growth factors (see Example 1).

In one Example of the present invention, cells were obtained by primary culture from temporal lobe, obtained from epilepsy patients by surgical operation. However, it is obvious to those skilled in the art that cells obtained from hippocampus tissue may be used in the present invention.

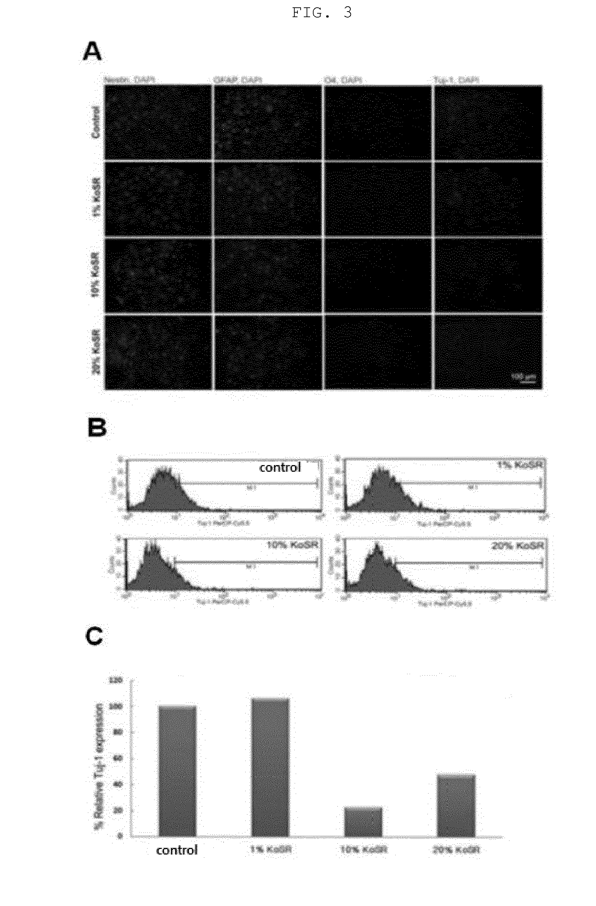

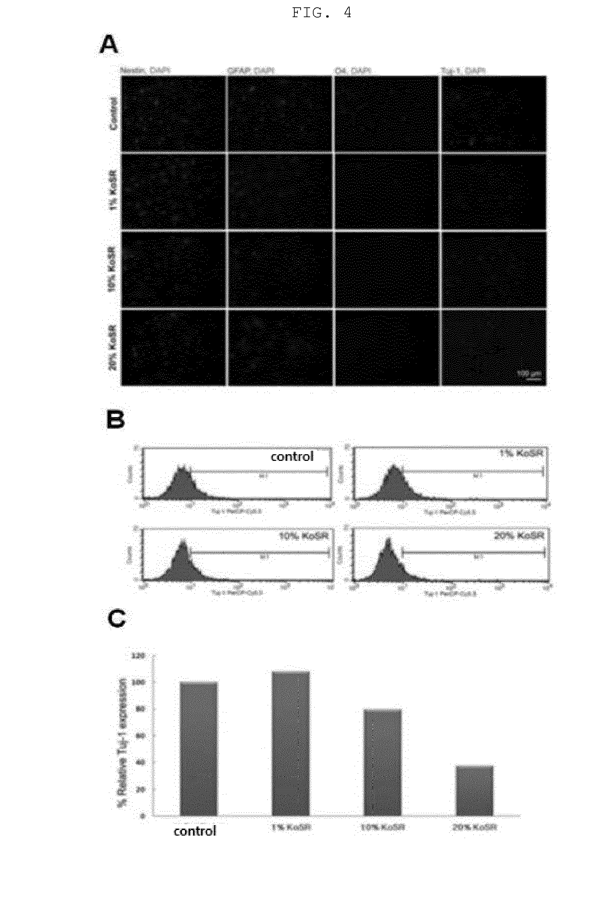

The present invention also provides human adult stem cells having a reduced ability to spontaneously differentiate. Neural stem cells cultured in the culture medium of the present invention have a reduced ability to spontaneously differentiate, and thus the use of the culture medium according to the present invention makes it possible to obtain a high purity of human adult neural stem cells (see FIGS. 3 and 4). Therefore, the present invention provides stem cells, preferably adult neural stem cells, which have a reduced ability to spontaneously differentiate. Most neural stem cells spontaneously differentiate regardless of the kind of tissue or cell from which these cells are derived, when these cells are cultured *in vitro* for a long period of time. As used herein, the term "spontaneous differentiation" is distinguished from guided differentiation and refers to a differentiation phenomenon that occurs even in a state in which artificial differentiation by treatment with external differentiation-inducible substances (growth factors, hormones, chemicals, etc.) is not induced. As used herein, the expression "high purity of stem cells" refers to stem cells having a low ability to spontaneously differentiate.

The following examples illustrate only the culture of human neural stem cells using the medium of the present invention, but it will be obvious to those skilled in the art that the culture of adult stem cells derived from other sources can also provide the same effect as that in the culture of human neural stem cells.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Preparation of human adult stem cells

### 1-1: Isolation and culture of human neural stem cells

Temporal lobe tissue was obtained from epilepsy patients (Department of Neurosurgery, Samsung Medical Center, Seoul, Korea) by surgical operation (cells obtained from patient 1 are referred to as cell #1, and cells obtained from patient 2 are referred to as cell #2). Within 3 hours after the surgical operation, each tissue was washed with PBS, and then mechanically cut using surgical scissors or blades. The cut tissue was treated at 37 °C for 1 hour or less with an enzymatic solution, prepared by mixing collagenase (0.4 mg/ml, Gibco), DNaseI (0.01-1 mg/ml, Roche), papain (10 unit/ml, Sigma), D-L-cystein (400 ng/ml, Sigma) and DNaseI (0.01-1 mg/ml, Roche). The treated tissue was dissociated to single cells using a serum pipette, and then passed through a nylon mesh, thereby obtaining single cells.

The single cell suspension was subjected to concentration gradient (Percoll, Sigma) and centrifuged to remove red blood cells and dead cells. The resulting cells were suspended in a Neurobasal-A (Gibco) medium or DMEM:F12 (Gibco) medium containing 1% FBS, 1X B27 (a dilution of a 50X concentration (Invitrogen)), N2 supplement (Gibco), 50 ng/ml bFGF (R&D) and 50 ng/ml EGF (R&D). Then, the cells were cultured in a cell culture plate pre-treated with poly-L-ornithine (Sigma), thereby obtaining primarily cultured neural stem cells.

### I-2 Analysis of characteristics of human neural stem cells

### Immunocytochemical staining

The neural stem cells obtained in Example 1-1 were fixed with 4% paraformaldehyde (PFA, Sigma) or acetone/methanol, and then permeabilized with PBS containing 0.05% Triton X-100 (Sigma) for 15 minutes. Then, the tissue was blocked with 5% normal horse serum/1% normal goat serum (Vector Lab.) at room temperature for 1 hour.

Next, the cells were washed several times with PBS containing 0.01% triton X-100 (Sigma) and treated with a combination of anti-CD133 (Abcam), anti-nestin (Abcam or Millipore), anti-Sox2 (R&D), anti-Hes3 (Santa Cruz), anti-GFAP (Sigma or Abcam), anti-Olig2 (Millipore), anti-04 (Chemicon) and anti-Tuj-I (Millipore). Then, the cells were incubated at 4 °C overnight.

Next, the cells were washed several times with PBS containing 0.01% triton X-100 and were treated with anti-mouse-488 (BD), anti-mouse-594 (BD), anti-rabbit-488 (BD), anti-rabbit-594 (BD), anti-rat-488 (BD) and anti-rat-594 (BD) secondary antibodies corresponding to the above primary antibodies. Finally, the cells were nucleus-stained with DAPI (Sigma), and the expression of final fluorescence was observed with a fluorescence microscope (Axiovert, Zeiss).

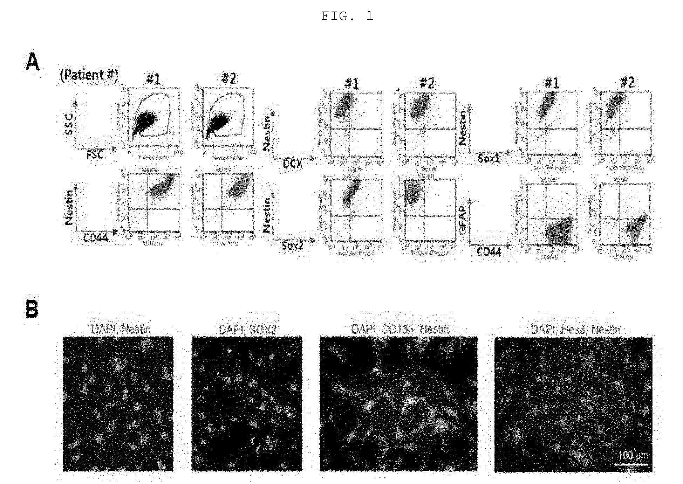

As a result, the expression of Nestin, Sox2, CD133 and Hes3 known as markers specific for neural stem cells could be observed (FIG. 1B).

### Flow cytometry analysis

The expression of neural stem cell-specific markers (Nestin, Sox1, and Sox2), a glial cell-specific marker (CD44), an astrocyte-specific marker (GFAP), a neuron-specific marker (DCX) and a cell proliferation marker (Ki-67) was analyzed by FACS.

The adult neural stem cells obtained in Example 1-1 were cultured by an adherent culture method, and then the characteristics of the neural stem cells were analyzed using a human neural lineage analysis kit (Catalog number: 561526) available from BD Biosceinces according to a standard experimental method provided by the manufacturer.

Specifically, the cultured cells were washed once with PBS, after which the cells were dissociated to single cells by treatment with accutase (Innovative Cell Technologies, Inc.), followed by centrifugation. Then, the cells were suspended in 2% FBS-containing FACS buffer and treated with BD Cytofix fixation buffer (Catalog number: 554655) and BD Phos flow perm buffer III (Catalog number: 558050), followed by treatment with a combination of CD44-FITC (Catalog number:555478), Ki67-AlexaFluor 488 (Catalog number: 561165), Doublecortin-PE (Catalog number: 561505), Sox1-PerCP-Cy5.5 (Catalog number: 561549), Sox2-PerCP-Cy5.5 (Catalog number: 561506), GFAP AlexaFluor (Catalog number: 561470) and Nestin-AlexaFluor (Catalog number: 561126) antibodies.

Next, the cells were washed once with FACS buffer, and then the characteristics of the cells were analyzed by flow cytometry (BD FACSCalibur).

As a result, it could be seen that Nestin known as a neural stem cell-specific marker protein and CD44 known as a glial cell-specific marker protein were expressed at a level of 99%, and Sox1 and Sox2, which are other markers specific for neural stem cells, were also expressed at a level of about 2-50%. In addition, it was shown that the astrocyte-specific marker protein GFAP and the neuron-specific marker protein DCX were expressed at relatively low levels (FIG. 1A).

### Example 2: Culture of cells in medium containing knockout serum replacement

Whether cells cultured in a medium (conventional medium) containing FBS and growth factors can be applied to the medium of the present invention was tested by suspending the human adult neural stem cells, obtained in Example 1-1, in a DMEM:F12 (Gibco) medium or Neurobasal-A (Gibco) medium containing B27 supplement (Gibco) and N2 supplement (Gibco), and then culturing the cells in a cell culture plate pre-treated with poly-L-ornithine (Sigma).

Specifically, the human adult neural stem cells obtained in Example 1-1 were cultured in a conventional medium, and the cultured cells were washed once with PBS, and then dissociated to single cells by treatment with accutase (Innovative Cell Technologies, Inc.), followed by centrifugation. The resulting cells were cultured in each of a conventional medium containing 1% FBS and growth factors and a medium containing 10% knockout serum replacement (see FIG. 2A).

As a result, it could be seen that, even when the human adult neural stem cells were cultured in the inventive medium containing no growth factor and FBS (the right photograph of FIG. 2B), these cells were cultured in a manner similar to the culture pattern in the conventional medium (the left photograph of FIG. 2B). This suggests that the cells cultured in the conventional medium are also cultured in the inventive medium in a similar manner. From such results, it could be seen that the use of the inventive medium containing the knockout serum replacement (KoSR) can effectively culture human adult neural stem cells, even when the medium does not contain expensive growth factors and the heterologous protein fetal bovine serum.

### Example 3: Culture of human adult neural stem cells in media containing various concentrations of knockout serum replacement

### Example 3-1: Culture of human adult neural stem cells in media treated with various concentrations of knockout serum replacement

The knockout serum replacement was suspended at various concentrations (1%, 10%, and 20%) in DMEM:F12 (Gibco) medium or a Neurobasal-A (Gibco) medium containing B27 supplement (Gibco) or N2 supplement (Gibco), and then the adult neural stem cells (#1 and #2) obtained in Example 1-1 were cultured in the media, thereby obtaining cultured neural stem cells. As a control, a 0.5% FBS-containing medium (containing no KoSR) was used.

### Example 3-2: Characteristics of human adult neural stem cells cultured in medium containing knockout serum replacement

### Immunocytochemical staining

Neural stem cells cultured in the inventive media containing various concentrations of the knockout serum replacement were subjected to immunocytochemical staining. Specifically, the neural stem cells obtained in Example 3-1 were fixed with 4% paraformaldehyde (Paraformaldehyde, PFA, Sigma) or acetone/methanol, and then permeabilized with PBS containing 0.05% Triton X-100 (Sigma) for 15 minutes. Then, the tissue was blocked with 5% normal horse serum/1% normal goat serum (Vector lab.) at room temperature for 1 hour.

Next, the cells were washed several times with PBS containing 0.01% triton X-100 (Sigma) and were treated with a combination of anti-nestin (Abcam or Millipore), anti-GFAP (Sigma or Abcam), anti-04 (Chemicon) and anti-Tuj-I (Millipore). Then, the cells were incubated at 4 °C overnight.

Next, the cells were washed several times with PBS containing 0.01% triton X-100 and were treated with a combination of anti-mouse-488 (BD), anti-mouse-594 (BD), anti-rabbit-488 (BD), anti-rabbit-594 (BD), anti-rat-488 (BD) and anti-rat-594(BD) secondary antibodies corresponding to the above primary antibodies. Finally, the cells were nucleus-stained with DAPI (Sigma), and the expression of final fluorescence was observed with a fluorescence microscope (Axiovert, Zeiss).

As a result, it could be seen that Nestin known as a neural stem cell-specific marker protein was strongly expressed (FIG. 3A and FIG. 4A).

### Flow cytometry analysis

The expression of a neural stem cell-specific marker protein (Nestin), an astrocyte-specific marker protein (GFAP), an oligodendrocyte-specific marker protein (04) and a neuron-specific marker protein (Tuj-1) was analyzed by FACS.

The characteristics of neural stem cells were analyzed using a human neural lineage analysis kit (Catalog number: 561526) available from BD Biosciences according to a standard experimental method provided by the manufacturer.

Specifically, the adult stem cells obtained in Example 3-1 were washed once with PBS, after which the cells were dissociated to single cells by treatment with accutase (Innovative Cell Technologies, Inc.), followed by centrifugation. The resulting cells were suspended in 2% FBS-containing FACS buffer. Then, the cells were treated with BD Cytofix fixation buffer (Catalog number: 554655) and BD Phos flow perm buffer III (Catalog number: 558050), and then treated antibody bound to PerCP-Cy5.5 fluorescence dye to a Tuj1 (Millipore, Catalog number: MAB1637). Next, the cells were washed once with FACS buffer, and then analyzed by flow cytometry (BD FACSCalibur).

As a result, it could be seen that the expression of Tuj1 known as a downstream neural cell-specific marker protein was expressed at a low level in the test cell group in the presence of the knockout serum replacement (FIGS. 3B and 4B).

### INDUSTRIAL APPLICABILITY

According to the present invention, a high-purity of stem cells can be cultured without having to use the heterologous protein fetal bovine serum or expensive growth factors (bFGF and EFG). Thus, stem cell therapy can be performed even at low costs without the risk of contamination by the heterologous protein.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A stem cell culture medium containing a basal medium and a knockout serum replacement (KoSR).

2. The stem cell culture medium of claim 1, wherein the basal comprises medium DMEM: F21, B27 supplement, and antibiotics.

3. The stem cell culture medium of claim 1, wherein the knockout serum replacement (KoSR) is contained in a concentration of 1-20% based on the stem cell culture medium.

4. The stem cell culture medium of claim 1, wherein the stem cell is an adult stem cell.

5. The stem cell culture medium of claim 4, wherein the stem cell is a neural stem cell or neural crest stem cell (NCSC) derived from a human adult brain tissue.

6. The stem cell culture medium of claim 5, wherein the brain tissue is a temporal lobe tissue or hippocampus tissue.

7. A method of culturing a stem cell having a reduced ability to spontaneously differentiate, the method comprising culturing stem cells in the culture medium of any one of claims 1 to 6.

8. The method of claim 7, wherein the stem cell is an adult stem cell.

9. The method of claim 8, wherein the stem cell is a neural stem cell or neural crest stem cell (NCSC) derived from a human adult brain tissue.

10. The method of claim 9, wherein the brain tissue is a temporal lobe tissue or hippocampus tissue.
